# FASCICULE DE BREVET EUROPEEN

(11) **EP 0 811 842 B1**
(45) Date de publication et mention de la délivrance du brevet: **19.05.1999**
(21) Numéro de dépôt: 97470014.8
(22) Date de dépôt: 02.06.1997
(51) Int. Cl.: G01N 33/18, G01N 21/64, G01N 21/76

(54) **Capteur biologique et procédé de surveillance de la qualité de l'eau**
Biosensor und Verfahren zur Überwachung der Wasserqualität
Biosensor and method for controlling the water quality

(30) Priorité: 03.06.1996 FR 9607016
(43) Date de publication de la demande: 10.12.1997
(73) Titulaire: Arnatronic Plus Sàrl, 54530 Arnaville (FR)
(72) Inventeur: Ory, Jean-Marie, 57000 Metz (FR)
(74) Mandataire: Schmit, Christian Norbert Marie

(56) Documents cités:
- DE-A- 2 626 915
- DE-A- 3 412 023
- DE-A- 4 334 327
- DE-C- 2 560 064
- US-A- 5 304 492
- ORY J -M ET AL: "A BIOSENSOR FOR WATER QUALITY MONITORING" JOINT PROCEEDINGS OF THE IEEE INSTRUMENTATION AND MEASUREMENT TECHNOLOGY CONFERENCE AND THE IMEKO TECHNICAL COMMITTEE 7, BRUSSELS, JUNE 4 - 6, 1996, vol. 2, 4 juin 1996, INSTITUTE OF ELECTRICAL AND ELECTRONICS ENGINEERS, pages 1354-1359, XP000618144

## Description

La présente invention concerne un capteur biologique de surveillance de la qualité de l'eau, ainsi qu'un procédé de mise en oeuvre d'un tel capteur, destiné à détecter la présence dans cette eau de traces de polluants tels que pesticides agricoles, résidus industriels, métaux lourds, nitrates, etc, en particulier pour la surveillance en continu du degré de pollution d'une eau en circulation, telle que par exemple l'eau courante d'une rivière.

On connaît déjà des méthodes et dispositifs destinés à la surveillance de la qualité de l'eau. Par exemple, il est connu de surveiller la qualité des eaux urbaines et de détecter d'éventuelles pollutions en observant le comportement d'animaux tels que poissons ou bactéries placés dans ces eaux, des changement de comportement étant révélateurs de modifications du milieu aquatique et notamment de la présence dans l'eau d'éléments polluants.

D'autres méthodes sont basées sur la détection de l'activité photosynthétique de plantes aquatiques ou d'algues ou, de manière générale, de végétaux chlorophylliens. On sait que la photosynthèse est le processus physico-chimique par lequel les plantes utilisent l'énergie de la lumière solaire pour convertir l'eau et le dioxyde de carbone en hydrates de carbone et en oxygène. L'activité photosynthétique d'une plante ou substance chlorophyllienne peut donc être évaluée par une mesure de la quantité d'oxygène généré par unité de temps, cette mesure donnant une indication sur le métabolisme global de la plante. Ainsi, on connaît déjà des procédés et dispositifs de détection de pollution des eaux basés sur ce principe et utilisant notamment certains types d'algues maintenues dans l'eau à surveiller, une mesure de l'oxygène généré par ces algues étant représentative de leur activité métabolique, laquelle est influencée par la présence de polluants dans l'eau.

On sait également qu'un végétal ré-émet une fluorescence dans le spectre lumineux rouge et proche infra-rouge (soit entre 650 et 800nm de longueur d'onde) lorsqu'il est éclairé. L'intensité de cette fluorescence est proportionnelle à la quantité de lumière reçue par la plante et qui n'a pas été assimilée pour la photosynthèse, suite à un blocage des transferts électroniques au niveau de la membrane des thylacoïdes. L'intensité de la fluorescence, qui est normalement faible lorsque le végétal est en bonne santé et exploite donc au maximum la lumière reçue pour la photosynthèse, peut augmenter dans de grandes proportions lorsqu'un facteur externe (produit chimique, variation brusque de température ou d'éclairement, etc) inhibe le métabolisme de la plante. La mesure de la fluorescence émise par des plantes ou algues maintenus dans un milieu aquatique est donc également représentative de la qualité du milieu où est placé la plante, et donc révélatrice de pollutions de ce milieu.

Les dispositifs connus mettant en oeuvre ces phénomènes et utilisant soit des mesures d'oxygène, soit des mesures de fluorescence sont actuellement essentiellement des appareils de laboratoire ou de test, nécessitant des prélèvements d'échantillons de l'eau surveillée (voir par exemple dans le document DE-A-4 334 327). Ils ne peuvent être utilisés pour effectuer des mesures en continu et sont donc inadaptés pour une surveillance continue de la qualité d'une eau courante et la détection de pollutions. Le dispositif décrit dans le document DE-A-2 626 915 ne permet pas la surveillance d'une eau circulante in-situ. En particulier, des pollutions fortes mais passagères et de courte durée risquent de ne pas pouvoir être détectées.

La présente invention a pour but de résoudre ces problèmes et vise à fournir un dispositif et un procédé de détection de pollution simple, permettant une surveillance continue de l'eau et une détection de pollutions durables ou passagères, notamment dans des eaux courantes telles que des eaux de rivières ou de circuits de distribution. Elle vise en particulier à permettre une surveillance de la qualité d'eaux courantes in-situ et à permettre l'enregistrement automatique et sur de longues durées des variations du degré de pollution de ces eaux courantes et la signalisation de brusques variations de ce degré de pollution.

Avec ces objectifs en vue, l'invention a pour objet un capteur biologique de surveillance de la qualité d'une eau circulante et de détection de pollution de cette eau, caractérisé en ce qu'il comporte :
- un élément de canalisation dans lequel l'eau peut circuler et dans la paroi duquel est ménagée une fenêtre dans laquelle sont placés :
- une paroi de support transparente,
- une membrane poreuse, sensiblement parallèle à la paroi de support, en communication directe avec l'intérieur de l'élément de canalisation et au travers de laquelle l'eau peut s'infiltrer,
- un biosubstrat chlorophyllien placé entre la paroi de support et la membrane poreuse,
- des moyens de mesure de l'activité métabolique du biosubstrat disposés contre la dite paroi de support, et
- une source lumineuse principale disposée de manière à éclairer le biosubstrat à travers la paroi de support transparente.

Le capteur selon l'invention est de construction simple et peut être aisément utilisé in-situ pour effectuer une détection en continu et en temps réel de pollution de l'eau conduite à travers l'élément de canalisation. En particulier et à titre d'exemple, dans le cas d'une surveillance des eaux urbaines circulant sous pression dans des conduites de distribution, le capteur peut être simplement raccordé sur ces conduites, via un robinet d'isolement. Dans le cas d'une surveillance des eaux de rivières par exemple, la circulation de l'eau dans le capteur pourra être assurée par une pompe péristaltique aspirant l'eau de la rivière et un circuit de pré-traitement en amont du capteur, assurant en particulier le filtrage de l'eau, le lissage du débit, le dégazage, la régulation de température, etc., et une conduite de rejet de l'eau après son passage dans le capteur.

On notera que, grâce à l'utilisation d'une source lumineuse éclairant le biosubstrat à travers la dite paroi de support, et donc située du même coté de cette paroi de support que les moyens de mesure, tous les éléments nécessitant une connexion à une source électrique ou à un circuit de traitement des signaux fournis par les dits moyens de mesure peuvent être regroupés dans un même boîtier. Ce boîtier peut également intégrer l'électronique de traitement des signaux de mesure, évitant ainsi les problèmes de parasitage qui perturberaient ces signaux s'ils étaient transmis par câble à une électronique de traitement éloignée du capteur.

Préférentiellement, afin de faciliter la maintenance du capteur et en particulier pour permettre un remplacement aisé du biosubstrat, l'élément de canalisation comporte en face de la dite fenêtre une ouverture obturée par un chapeau étanche et facilement démontable, qui fournit un accès facile à la membrane poreuse et au biosubstrat.

L'invention a aussi pour objet un procédé de détection de la pollution d'une eau circulante caractérisé en ce qu'on place dans une fenêtre ménagée dans la paroi d'un élément de canalisation traversé par la dite eau un biosubstrat chlorophyllien, maintenu entre une membrane poreuse en communication directe avec l'intérieur de l'élément de canalisation et une paroi de support transparente, on dispose du coté de la dite paroi de support opposé à l'intérieur de la canalisation des moyens de mesure de l'activité métabolique du biosubstrat, on éclaire le dit biosubstrat à travers la dite paroi de support, et on mesure en permanence l'activité métabolique du biosubstrat, les variations des signaux fournis par les dits moyens de mesure étant représentatives de l'état de pollution de l'eau.

La mesure de l'activité métabolique du biosubstrat peut être réalisée soit par mesure de la quantité d'oxygéne provenant de la photosynthèse du biosubstrat, soit par mesure de la fluorescence chlorophyllienne.

Dans le premier cas, les moyens de mesures sont préférentiellement constitués par un dispositif de type électrode de CLARK, mesurant la quantité d'oxygène dissous passant à travers la paroi de support, constituée alors d'un matériau perméable à l'oxygène mais imperméable à l'eau, tel que un film de PTFE. L'éclairage est appliqué par intermittence avec une période d'environ 1 minute par exemple (20 secondes d'éclairement, 40 secondes d'extinction). Dans ces conditions, on enregistre en continu la dérivée du signal de concentration d'oxygène fourni par les moyens de mesure, qui reflète l'activité photosynthétique du biosubstrat et permet donc de détecter la présence dans l'eau d'éléments polluants.

Dans le second cas, les moyens de mesure sont constitués par un détecteur de lumière tel qu'une photodiode qui mesure, dans la gamme du spectre lumineux rouge ou proche de l'infrarouge (préférentiellement des longueurs d'onde supérieures à 650 nm), la fluorescence émise par le biosubstrat en réponse à une illumination modulée de ce biosubstrat, générée par une source lumineuse complémentaire, émettant par exemple sous une longueur d'onde de 470 nm. La modulation de l'illumination induit une fluorescence modulée du substrat chlorophyllien qui est détectée par la photodiode. Une démodulation synchrone du signal amplifié de la photodiode permet de s'affranchir de l'important bruit du signal généré par la photodiode en ne conservant que les éléments de ce signal correspondants à la modulation de la source lumineuse complémentaire. L'éclairage principal peut être commandé de manière à simuler des conditions d'éclairement naturel du jour ou de la nuit, ce qui permet de faire fonctionner la substance chlorophyllienne selon les deux modes métaboliques du cycle de Calvin. La source de lumière principale peut également être utilisée en mode pulsé pour provoquer des transitoires connus sous le nom de courbe de Kautski (qui représente la réponse en fluorescence d'un végétal à des variations brusques d'éclairement), et l'on détermine alors les paramètres caractéristiques de la courbe obtenue afin d'en déduire l'état métabolique du biosubstrat.

D'autres caractéristiques et avantages apparaîtront dans la description qui va être faite, à titre d'exemple, de deux modes de réalisation de l'invention, un premier mode étant basé sur la mesure de la fluorescence chlorophyllienne, le deuxième mode étant basé sur la mesure de la quantité d'oxygène généré par la photosynthèse.

On se reportera aux dessins annexés dans lesquels :
- la figure 1 est une vue en coupe d'un capteur correspondant au premier mode ;
- la figure 2 représente un capteur destiné au second mode.

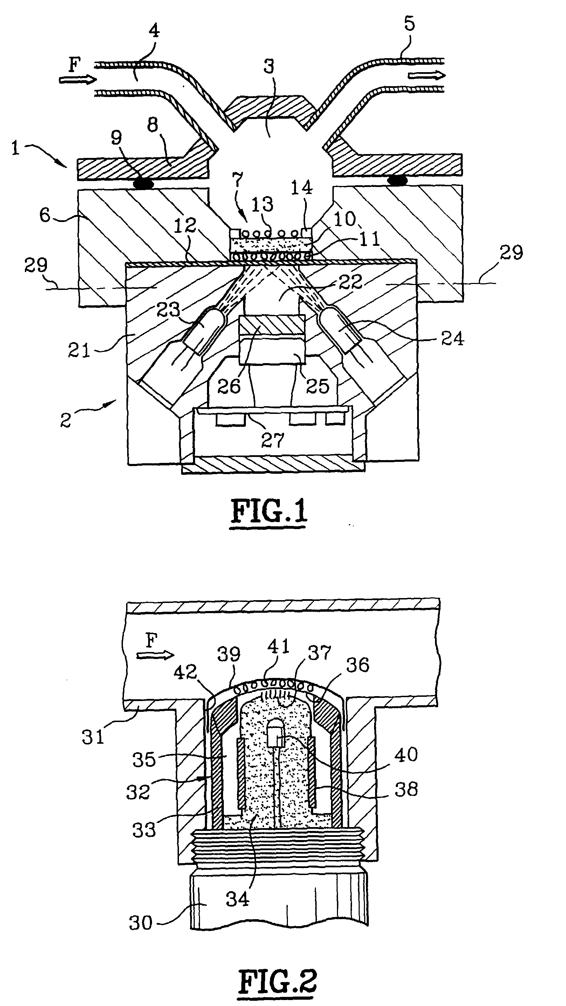

Le capteur représenté figure 1 comporte un corps 1 constituant un élément de canalisation dans lequel l'eau à analyser circule selon les flèches F, et un fluorimètre 2 fixé sur le corps 1 de capteur à l'aide de vis de blocage, représentées seulement par leurs axes 29 (par exemple, trois vis disposées radialement à 120° suffisent à bloquer le fluorimètre sur le corps 1, tout en permettant de les séparer très facilement). Le corps 1 est préférentiellement réalisé en un matériau synthétique opaque, de préférence de couleur noire, par exemple en PVC ou Delrin. Le corps 1 comporte une chambre interne 3, ayant par exemple une section de forme polygonale, dans laquelle débouche un conduit 4 d'entrée d'eau et un conduit de sortie 5.

Le corps 1 est préférentiellement formé d'une base 6 dans la paroi de laquelle est ménagée une fenêtre 7 débouchant dans la chambre 3, et d'un chapeau 8 démontable sur lequel sont raccordés les conduits d'entrée et de sortie 4 et 5. Le chapeau 8 est maintenu sur la base 6 par un système de fixation à démontage rapide de type connu en soi, par exemple du type couvercle de bocal à conserves. L'étanchéité entre la base et le chapeau est assurée par exemple par un joint torique 9.

Dans la fenêtre 7 est disposé un filtre microporeux 10, perméable à l'eau, par exemple en nitrate de cellulose, sur lequel est déposé en couche mince, du coté opposé à la chambre 3, un biosubstrat chlorophyllien 11, maintenu par ailleurs en contact avec une paroi de support formée d'un film transparent 12 chimiquement inerte, ou d'une lame de verre, maintenu de manière étanche entre le corps 1 et le fluorimètre 2, par exemple par collage sur le corps 1.

Une rondelle 13 formée d'un grillage rigide en un matériau inerte est bloquée dans la fenêtre 7 par une bague élastique 14 et permet d'assurer une pression sur le filtre microporeux 10 de manière à immobiliser le biosubstrat entre le dit filtre et le film transparent 12.

Le biosubstrat est préférentiellement constitué d'une couche d'algues microscopiques, mais il peut également être constitué de diatomées, cyanobactéries, lichens, thylacoïdes extraits d'un végétal quelconque, ou toute autre forme de système chlorophyllien, y compris des plantes supérieures ou des parties de celles-ci. Un gel tel que l'Alginate sous forme de couche mince ou de micro-billes peut également être utilisé pour fixer le biosubstrat.

La chambre 3 et destinée à créer de fortes turbulences de l'eau pour assurer une bonne diffusion des molécules de polluant contenues dans l'eau au travers du filtre 10, et assurer ainsi une détection efficace grâce à un renouvellement rapide de l'eau au contact du biosubstrat. Le chapeau démontable 8 permet de remplacer régulièrement, aisément et rapidement le biosubstrat lors des opérations de maintenance.

Le fluorimètre 2 est constitué d'un boîtier 21, par exemple métallique, en aluminium ou dural, comportant une ouverture centrale 22 débouchant sur le film 12 en face du biosubstrat 11. Il comporte une source lumineuse principale formée par exemple par plusieurs puissantes diodes luminescentes 23 encastrées dans le boîtier sur le coté de l'ouverture 22 (par exemple quatre diodes réparties autour de l'ouverture 22) et disposées de manière que le faisceau lumineux émis soit dirigé sur le biosubstrat 11. Préférentiellement, ces diodes 23 sont des diodes jaunes-ambre fournissant une lumière d'environ 590 nm de longueur d'onde, et permettant de simuler, selon qu'elles sont éteintes ou allumées, des conditions d'éclairement du jour ou de la nuit, pour mettre le biosubstrat dans des conditions correspondant aux deux modes métaboliques du cycle de Calvin. L'intensité lumineuse fournie par ces diodes pourra par exemple être de 6 candelas ou plus.

Une ou plusieurs autres diodes, destinées spécifiquement à générer la fluorescence du biosubstrat, par exemple des diodes bleues 24 de faible intensité émettant à 470 nm de longueur d'onde, sont également disposées dans le boîtier, de manière similaire aux diodes 23 ( par exemple quatre diodes bleues réparties entre les diodes ambres 23).

Une photodiode 25, devant laquelle est placé un filtre optique 26 arrêtant les longueurs d'ondes inférieures à environ 650 nm, est disposée axialement dans l'ouverture 22, pour détecter la fluorescence du biosubstrat, et reliée à un circuit électronique de pré-amplification 27, intégré également dans le boîtier métallique 21, de manière à assurer une bonne immunité de ce circuit aux parasites. Un autre circuit électronique, qui peut également être placé dans le boîtier 32, assure l'alimentation des diodes luminescentes, et en particulier pilote les phases d'éclairement et d'extinction des diodes jaunes-ambres ainsi que la modulation des diodes bleues, utile, comme on le verra par la suite, pour améliorer le traitement du signal fourni par la photodiode 25. Compte tenu que le signal de fluorescence fourni par la photodiode 25 est très faible, le circuit d'alimentation des diodes 23 et 24 est préférentiellement complètement séparé du circuit de la photodiode 25 et blindé pour éviter de parasiter ce dernier.

Le circuit de pré-amplification est par ailleurs relié à un dispositif électronique de traitement, non représenté, constitué par exemple par un processeur de signal DSP56001 associé à des convertisseurs analogique-numérique et un port de communication vers un ordinateur, qui permet d'obtenir le niveau de fluorescence par démodulation synchrone du signal amplifié de la photodiode.

A la place des diodes luminescentes 23, 24 de différentes couleurs, on pourrait également utiliser des diodes laser ou, pour la source de lumière principale, une source halogène. De même, la photodiode 25 pourrait, dans un mode de réalisation plus couteux, être avantageusement remplacée par un photomultiplicateur sensible dans le rouge. La disposition des diodes luminescentes pourrait également être modifiée, par exemple, de manière que leur rayonnement soit dirigé le plus près possible de la direction perpendiculaire à la paroi de support 12, pour éviter les réflexions parasites et diminuer le plus possible la lumière perdue par réflexion. On pourrait alors utiliser une photo-cellule de moindre dimension, ou éloigner celle-ci de la paroi de support et y ramener la lumière fluorescente à l'aide d'un conduit de lumière ou un faisceau de fibres optiques.

Lors de la mise en oeuvre du capteur, l'eau à analyser est amenée dans la chambre 3, soit en raccordant directement le conduit d'entrée 4 sur une conduite sous pression, par exemple dans le cas d'analyse d'eaux urbaines, soit par une pompe et un circuit de filtration dans le cas d'eau courante, par exemple de rivière. Avantageusement, des moyens de régulation en température seront disposés en amont de la chambre 3. On pourra notamment utiliser pour cela des éléments à effet Pelletier, intégrés par exemple dans le conduit d'entrée 4 du capteur. On veillera également à ce que le débit d'eau traversant le capteur soit suffisamment important, pour assurer le renouvellement rapide de l'eau baignant le biosubstrat et un brassage suffisant pour garantir une bonne diffusion des traces de polluants à travers le filtre poreux. On notera par ailleurs que la disposition du capteur représentée sur la figure, c'est à dire avec la fenêtre 7 située vers le bas de la chambre 3 permet d'éviter que des bulles ne viennent en contact avec le biosubstrat (ceci étant particulièrement important dans le cas de la deuxième variante utilisant une mesure d'oxygène dissous, qui sera décrite par la suite).

L'illumination du biosubstrat est réalisée par les diodes jaunes-ambre 23, comme indiqué préalablement, qui servent à générer le processus de photosynthèse. On notera à ce sujet que l'opacité du matériau du corps de capteur permet d'éviter toute influence parasite d'un éclairement extérieur aléatoire. Ces diodes peuvent également être utilisées en mode pulsé, avec une période de l'ordre de 15 secondes par exemple, pour générer les courbes de Kautski déjà mentionnées. Ces courbes se caractérisent par une brusque augmentation de la fluorescence d'un végétal, tel que par exemple des algues de l'espèce "senedesmus", en réponse à un passage d'une obscurité maintenue suffisamment longtemps à un éclairement suffisamment intense. En présence d'eau propre, cette fluorescence décroît rapidement en quelques secondes ; par contre, en présence d'un polluant tel que l'Atrazine, cette fluorescence reste durablement à un niveau élevé. Ce mode d'éclairement pulsé permet donc de détecter et caractériser des polluants en fonction de leur effet sur le biosubstrat, par une analyse des caractéristiques des courbes de Kautski générées par traitement des signaux issus de la photodiode.

La mesure proprement dite de la fluorescence est effectuée par la photodiode, qui capte le rayonnement fluorescent issu du biosubstrat. La modulation du rayonnement fourni par les diodes bleues 24 ( sous une fréquence de l'ordre du kHz) permet d'améliorer fortement la détection, car le signal de fluorescence est très faible et noyé dans le bruit global des signaux fournis par la photodiode. En extrayant, par une démodulation synchrone, du signal pré-amplifié de la photodiode les signaux correspondant à la fréquence de modulation des diodes bleues, on ne conserve alors que les signaux représentatifs de la fluorescence du biosubstrat modulée en accord avec la modulation de la source lumineuse. Par ailleurs, le filtre optique 26 étant quasiment parfaitement opaque au rayonnement des diodes luminescentes, la photodiode ne reçoit que les composantes du rayonnement correspondant à la fluorescence modulée, de longueur d'onde supérieure à 650 nm, à l'exclusion des rayonnements parasites provenant des sources lumineuses. On notera à ce sujet que l'utilisation d'un rayonnement de longueur d'onde voisine de 470 nm est particulièrement intéressant car ce rayonnement procure un rendement de fluorescence maximal du biosubstrat. On notera également que le but essentiel du filtre optique 26 et d'empêcher que le rayonnement modulé des diodes bleues 24 parvienne à la photodiode, afin que, après la démodulation synchrone, le signal obtenu reflète uniquement la fluorescence chlorophyllienne. Il sert également à éviter que la photodiode reçoive un excès de lumière provenant de la source d'éclairage principal, susceptible de masquer le signal très faible de fluorescence. Pour améliorer encore la sensibilité de détection, on pourrait aussi filtrer la lumière émise par les diodes luminescentes avec un filtre ne passant pas les longueurs d'ondes supérieures à 650 nm, ou utiliser des sources de lumière polarisée pour éviter les réflexions des rayonnements autres que ceux de fluorescence.

Après le traitement de démodulation synchrone, le signal obtenu, représentatif en continu de la fluorescence chlorophyllienne du biosubstrat, peut subir de nombreux autres traitements ultérieurs aboutissant à l'affichage d'un graphique daté dans lequel les variations infimes de fluorescence et les dates de ces variations apparaissent clairement.

A titre d'exemple des résultats obtenus grâce au procédé et au capteur décrits précédemment, il a été possible de détecter de manière fiable des traces d'Atrazine ou de Diuron présentes dans l'eau à une concentration inférieure à 1 µg par litre.

Le dessin de la figure 2 représente un deuxième mode de réalisation du capteur, basé sur la mesure de l'oxygène généré par la photosynthèse du biosubstrat. Le capteur comporte un boîtier 30 fixé sur un élément de canalisation 31. Le boîtier 30 porte un détecteur 32 d'oxygène dissous, constitué de manière similaire à une électrode de Clark modifiée. Ce détecteur comporte une enveloppe opaque 33 de forme générale cylindrique, par exemple en PVC ou Delrin, à l'intérieur de laquelle est disposée coaxialement un porte-électrode 34 également cylindrique, par exemple en PVC transparent, dans lequel sont placées une ou plusieurs diodes luminescentes 40. Une chambre annulaire 35, ménagée entre le porte-électrode et l'enveloppe est remplie d'un électrolyte tel que KCl. L'enveloppe 33 est fermée à son extrémité par une paroi transparente à la lumière et perméable exclusivement à l'oxygène, telle qu'une membrane en PTFE 36. Le porte-électrode 34 s'étend jusqu'à proximité de cette membrane et porte sur sa paroi frontale une cathode 37, par exemple en or, formée d'une grille ou d'une spirale ou encore d'une feuille très mince d'or, de manière que cette cathode soit transparente à la lumière. Une anode 38 en argent est placée sur la paroi latérale du porte-électrode.

Une membrane poreuse 39 sur laquelle est déposée en couche mince un biosubstrat chlorophyllien 41 est fixée sur l'enveloppe 33 de manière à presser fermement le biosubstrat 41 contre le film de PTFE 36. Les dimensions du détecteur sont déterminées de manière que la membrane poreuse 39 soit sensiblement affleurante à la surface interne de la paroi de l'élément de canalisation, dans une fenêtre 42 ménagée à cet effet dans cette paroi.

Lors de la mise en oeuvre de ce capteur, l'eau circulant dans l'élément de canalisation 31 baigne le biosubstrat 41 en traversant la membrane poreuse 39. L'activité photosynthétique du biosubstrat, générée par l'éclairage procuré par les diodes 40, génère de l'oxygène qui traverse le film de PTFE 36 et se retrouve dissous dans l'électrolyte 35. La concentration d'oxygéne ainsi générée est déterminée par la mesure de l'intensité du courant circulant entre l'anode 38 et la cathode 37, entre lesquelles est maintenue une tension de polarisation de l'ordre de 0,7 volts. La courbe représentative du signal obtenu en fonction du temps représente donc, aux pertes près, l'intégrale de la quantité d'oxygène généré. Afin de pouvoir déterminer les variations dans le temps de la quantité d'oxygène généré par photosynthèse, la diode est alimentée cycliquement, de sorte que, bien que croissant, le signal obtenu reflète les cycles d'éclairement. Par dérivation, on obtient alors un signal représentatif de la quantité d'oxygène généré par unité de temps et donc de l'activité photosynthétique du biosubstrat. Afin de limiter l'influence des variations de température, d'une part la température de l'eau sera régulée autant que possible en aval du capteur, comme indiqué pour le premier mode de réalisation, et d'autre part un capteur de température pourra être intégré dans le détecteur, par exemple dans le porte-électrode, et la valeur mesurée prise en compte dans le traitement du signal représentatif de la quantité d'oxygène généré.

Ce dispositif a permis de détecter efficacement des traces de divers polluants, par exemple la présence d'Atrazine en concentration de 0,4 mg/litre, d'ions bichromate à 1 ppm, cyanure à 1 ppm, chlore à 3,6 mg/l, ou encore de phosphates ou nitrates, l'efficacité de détection étant cependant dépendante de la nature du biosubstrat utilisé. Le seuil de sensibilité actuellement atteint est de l'ordre de 50 µg/l.

Quelque soit la méthode de mesure utilisée, il est particulièrement important que l'eau soit bien brassée pour assurer l'homogénéisation de la température et la diffusion des polluants dans le biosubstrat. Pour cela, on utilisera avantageusement une pompe, par exemple une pompe centrifuge, qui permettra de plus d'augmenter la vitesse de l'eau dans la chambre 3, pour faire circuler l'eau en circuit bouclé, en passant au travers d'un réservoir tampon et d'un bloc de régulation de température.

Dans une utilisation en laboratoire, le circuit peut être fermé, de volume connu, par exemple de un litre, le polluant étant introduit en quantité déterminée dans ce circuit.

Pour une utilisation en surveillance continue, une difficulté est de maintenir une température constante de l'eau sans dépenser de fortes quantités d'énergie. Pour cela, plusieurs méthodes peuvent être utilisées :
- procéder à un mélange progressif, en utilisant une pompe péristaltique pour injecter en permanence dans le circuit bouclé un petit débit, par exemple quelques cm₃/mn, de l'eau à tester, pendant que la même quantité d'eau est rejetée ;
- ou procéder par échantillonnage, en prélevant lentement un volume d'eau à tester identique au volume du circuit fermé, et préchauffer cette eau pendant que se réalise la mesure sur l'échantillon précédent ; puis un mécanisme d'électrovannes substitue cette eau préchauffée au circuit principal pendant que l'échantillon précédent est rapidement rejeté. Idéalement, la durée du prélèvement est identique à celle de la mesure ; ainsi on ne perd pas d'informations. Un échangeur thermique peut servir à réchauffer ou refroidir l'eau prélevée par l'eau rejetée.

Dans le cas d'un fonctionnement par échantillonnage, plusieurs circuits d'eau ou plusieurs capteurs complets correspondant à des instants décalés dans le temps pourraient être utilisés en simultanéité, dans le but de diminuer les temps de réponse.

Dans le cas où l'on souhaite une mesure précise et fine, il est préférable de travailler par comparaison. Cela nécessite une ou plusieurs eaux de référence que l'on pourra injecter dans le capteur à des instants donnés, de manière à le calibrer ou recaler son zéro. Le processus nécessite un préchauffage comme dans le cas de l'échantillonnage.

Pour éviter l'influence de la présence incontrôlée de gaz dissous dans l'eau, on procédera préférentiellement, en amont du dispositif, à un vigoureux brassage ou bullage avec de l'air, à température d'eau régulée, permettant d'uniformiser la quantité de gaz dissous.

## Revendications

1. Capteur biologique de surveillance de la qualité d'une eau circulante et de détection de pollution de cette eau, caractérisé en ce qu'il comporte :
- un élément de canalisation (1, 31) dans lequel la dite eau peut circuler et dans la paroi duquel est ménagée une fenêtre (7, 42) dans laquelle sont placés :
- une paroi de support (12, 36) transparente,
- une membrane poreuse (10, 39), sensiblement parallèle à la paroi de support, en communication directe avec l'intérieur de l'élément de canalisation et au travers de laquelle l'eau peut s'infiltrer,
- un biosubstrat chlorophyllien (11, 41) placé entre la paroi de support et la membrane poreuse,
- des moyens (2 ; 32) de mesure de l'activité métabolique du biosubstrat disposés contre la dite paroi de support, et
- une source lumineuse principale (23, 40) disposée de manière à éclairer le biosubstrat à travers la paroi de support.

2. Capteur biologique selon la revendication 1, caractérisé en ce que l'élément de canalisation (1) comporte en face de la dite fenêtre (7) une ouverture obturée par un chapeau (8) étanche et facilement démontable.

3. Capteur biologique selon la revendication 1, caractérisé en ce que la dite fenêtre est réalisée dans la paroi d'une chambre (3) dans laquelle débouchent un conduit d'entrée (4) et un conduit de sortie (5) de l'eau.

4. Capteur biologique selon la revendication 1, caractérisé en ce que les moyens de mesure sont des moyens de mesure de la fluorescence chlorophyllienne du biosubstrat.

5. Capteur biologique selon la revendication 3, caractérisé en ce que les dits moyens de mesure comportent un boîtier (21) pourvu d'une ouverture (22) disposée face à la fenêtre (7), un détecteur de lumière (25) placé dans la dite ouverture et pourvu d'un filtre optique (26), pour mesurer la fluorescence du biosubstrat, une source lumineuse complémentaire (24) pour créer une illumination modulée du biosubstrat, et des moyens de traitement pour effectuer une démodulation synchrone du signal fourni par le détecteur.

6. Capteur biologique selon la revendication 3, caractérisé en ce que la source lumineuse principale est constituée de diodes luminescentes (23) placées dans le boîtier (21) et émettant à une longueur d'onde voisine de 550 nm, la source lumineuse complémentaire est formée de diodes émettant à une longueur d'onde voisine de 470 nm, les diodes étant disposées de manière que leur rayonnement soit dirigé vers le biosubstrat (11), et le filtre optique (26) est un filtre passant les longueurs d'ondes supérieures à environ 650 nm.

7. Capteur biologique selon la revendication 1, caractérisé en ce que la paroi de support (36) est en un matériau perméable à l'oxygéne mais imperméable à l'eau, et les moyens de mesure sont des moyens de mesure (32) de la quantité d'oxygène généré par la photosynthèse du biosubstrat et traversant la dite paroi de support, les dits moyens de mesure (32) comportant une enveloppe (33) entourant un porte-électrode (34) et ménageant entr'eux un espace annulaire (35) rempli d'électrolyte, le porte-électrode portant latéralement une anode (38) et, sur sa face frontale située à proximité de la paroi de support (36), une cathode (37) transparente aux rayonnements lumineux, la source lumineuse (40) étant située dans le dit porte-électrode.

8. Procédé de détection de la pollution d'une eau circulante caractérisé en ce qu'on place dans une fenêtre (7, 42) ménagée dans la paroi d'un élément de canalisation (1, 31) traversé par la dite eau un biosubstrat chlorophyllien (11, 41), maintenu entre une membrane poreuse (10, 39) en communication directe avec l'intérieur de l'élément de canalisation et une paroi de support transparente (12, 36), on dispose du coté de la dite paroi de support opposé à l'intérieur de la canalisation des moyens (2, 32) de mesure de l'activité métabolique du biosubstrat, on éclaire le dit biosubstrat à travers la dite paroi de support, et on mesure en permanence l'activité métabolique du biosubstrat, les variations des signaux fournis par les dits moyens de mesure étant représentatives de l'état de pollution de l'eau.

9. Procédé selon la revendication 8 caractérisé en ce que on éclaire le biosubstrat d'une part par un rayonnement de longueur d'onde voisine de 550 nm dans des conditions simulant un éclairement naturel, et d'autre part par un rayonnement modulé dans le temps de longueur d'onde voisine de 470 nm, de manière à induire une fluorescence modulée du biosubstrat, on mesure cette fluorescence par un détecteur de lumière (25) pourvu d'un filtre (26) arrêtant les longueurs d'onde inférieures à environ 650 nm, et on procède à une démodulation synchrone du signal fourni par le détecteur (25) de manière à déterminer le niveau de fluorescence du biosubstrat, lequel est représentatif de son activité métabolique.

10. Procédé selon la revendication 8 caractérisé en ce que la paroi de support est réalisée en un matériau perméable à l'oxygène mais imperméable à l'eau, et, pour déterminer l'activité métabolique du biosubstrat, on mesure les variations dans le temps de la quantité d'oxygène généré par la photosynthèse du biosubstrat et traversant la dite paroi de support.

## Patentansprüche

1. Biosensor zur Überwachung der Qualität von fließendem Wasser und zum Nachweis einer Verschmutzung dieses Wassers, dadurch gekennzeichnet, daß er umfaßt:
- eine Durchflußvorrichtung (1, 31), durch die das Wasser geleitet fließen kann und in deren Wandung ein Fenster (7, 42) vorgesehen ist, in der angeordnet sind:
- eine durchsichtige Trägerwand (12, 36),
- eine poröse Membran (10, 39), die im wesentlichen parallel zur Trägerwand angeordnet ist, die in unmittelbarem Kontakt mit dem Innenraum der Durchflußvorrichtung steht und durch die das Wasser hindurchtreten kann,
- ein chlorophyllhaltiges Biosubstrat (11, 41), das zwischen der Trägerwand und der porösen Membran angeordnet ist,
- eine Meßeinrichtung (2; 32) zur Messung der metabolischen Aktivität des auf der Trägerwand angeordneten Biosubstrats, und
- eine Hauptlichtquelle (23, 40), die so angeordnet ist, daß das Biosubstrat durch die Trägerwand hindurch beleuchtet wird.

2. Biosensor nach Anspruch 1, dadurch gekennzeichnet, daß die Durchflußvorrichtung (1) dem Fenster (7) gegenüber eine Öffnung aufweist, die durch eine dicht schließende und leicht abnehmbare Abdeckung verschlossen ist.

3. Biosensor nach Anspruch 1, dadurch gekennzeichnet, daß das Fenster in der Wandung einer Kammer (3) ausgebildet ist, in die eine Wasserzuleitung (4) und eine Wasserableitung (5) münden.

4. Biosensor nach Anspruch 1, dadurch gekennzeichnet, daß die Meßeinrichtung eine Einrichtung zur Messung der Fluoreszenz des Chlorophylls des Biosubstrats ist.

5. Biosensor nach Anspruch 3, dadurch gekennzeichnet, daß die Meßeinrichtung umfaßt:
ein Gehäuse (21), das mit einer Öffnung (22) versehen ist, die dem Fenster (7) gegenüber angeordnet ist,
einen Lichtdetektor (25), der in dieser Öffnung angeordnet ist und mit einem optischen Filter (26) versehen ist, zur Messung der Fluoreszenz des Biosubstrats,
eine zusätzliche Lichtquelle (24) zur Erzeugung einer modulierten Beleuchtung des Biosubstrats und
eine Verarbeitungseinrichtung zur Durchführung einer synchronen Demodulation des von dem Detektor gelieferten Signals.

6. Biosensor nach Anspruch 3, dadurch gekennzeichnet, daß die Hauptlichtquelle aus Leuchtdioden (23) besteht, die in dem Gehäuse (21) angeordnet sind und Licht mit einer Wellenlänge von etwa 550 nm aussenden, daß die zusätzliche Lichtquelle aus Dioden gebildet ist, die Licht mit einer Wellenlänge von etwa 470 nm aussenden, wobei die Dioden so angeordnet sind, daß die Strahlung auf das Biosubstrat (11) gerichtet ist, und daß das optische Filter (26) ein Filter ist, das für Licht mit einer Wellenlänge oberhalb etwa 650 nm durchlässig ist.

7. Biosensor nach Anspruch 1, dadurch gekennzeichnet, daß die Trägerwand (36) aus einem Material besteht, das durchlässig für Sauerstoff, aber undurchlässig für Wasser ist, und daß die Meßeinrichtung eine Einrichtung (32) zur Messung der vom Biosubstrat durch Photosynthese gebildeten und durch die Trägerwand hindurchtretenden Sauerstoffmenge ist, wobei die Meßeinrichtung (32) eine Hülle (33) umfaßt, die einen Elektrodenhalter (34) umgibt und von dem Elektrodenhalter durch einen ringförmigen Zwischenraum beabstandet ist, der mit einem Elektrolyt gefüllt ist, wobei der Elektrodenträger seitlich angeordnet eine Anode (38) und auf der Stirnfläche, die in Nähe der Trägerwand (36) gelegen ist, eine für Lichtstrahlung durchlässige Kathode (37) aufweist, wobei die Lichtquelle (40) in dem Elektrodenträger angeordnet ist.

8. Verfahren zum Nachweis der Verschmutzung von fließendem Wasser, dadurch gekennzeichnet, daß in einem Fenster (7, 42), das in der Wandung einer Durchflußvorrichtung (1, 31), durch die das Wasser hindurchfließt, vorgesehen ist, ein chlorophyllhaltiges Biosubstrat (11, 41) angeordnet wird, das zwischen einer porösen Membran (10, 39), die sich in unmittelbarer Verbindung mit dem Innenraum der Durchflußvorrichtung befindet, und einer durchsichtigen Trägerwand (12, 36) gehalten wird, daß auf der dem Innenraum der Durchflußleitung gegenüberliegenden Seite eine Meßeinrichtung (2, 32) zur Messung der metabolischen Aktivität des Biosubstrats angeordnet wird, daß das Biosubstrat durch die Trägerwand hindurch beleuchtet wird und daß die metabolische Aktivität des Biosubstrats kontinuierlich gemessen wird, wobei die Änderungen der von der Meßeinrichtung gelieferten Signale für den Verschmutzungszustand des Wassers charakteristisch sind.

9. Verfahren nach Anspruch 8, dadurch gekennzeichnet, daß das Biosubstrat einerseits mit einer Strahlung mit einer Wellenlänge von etwa 550 nm unter Bedingungen, die eine natürliche Beleuchtung nachbilden, und andererseits mit einer zeitabhängig modulierten Strahlung mit einer Wellenlänge von etwa 470 nm beleuchtet wird, um eine modulierte Fluoreszenz des Biosubstrats zu erzeugen, daß diese Fluoreszenz mit einem Lichtdetektor (25) nachgewiesen wird, der mit einem Filter ausgestattet ist, das für Licht mit einer Wellenlänge unter etwa 650 nm nicht durchlässig ist, und daß eine synchrone Demodulation des von dem Detektor (25) gelieferten Signals durchgeführt wird, um das Ausmaß der Fluoreszenz des Biosubstrats zu bestimmen, das für die metabolische Aktivität des Biosubstrats charakteristisch ist.

10. Verfahren nach Anspruch 8, dadurch gekennzeichnet, daß die Trägerwand aus einem Material erzeugt wird, das für Sauerstoff durchlässig und für Wasser undurchlässig ist, und daß die zeitliche Änderung der durch die Photosynthese des Biosubstrats gebildeten und durch die Trägerwand hindurchtretenden Sauerstoffmenge gemessen wird.

## Claims

1. Biological sensor for monitoring the quality of a circulating flow of water and for detecting pollution in this flow of water, characterised in that it comprises:
- a channelling means (1, 31) in which said water can circulate and in the wall of which a window (7, 42) is disposed in which the following are located:
- a transparent supporting wall (12, 36),
- a porous membrane (10, 39), which is essentially parallel to the supporting wall, communicates directly with the interior of the channelling means and through which the water can infiltrate,
- a chlorophyll biosubstrate (11, 41) positioned between the supporting wall and the porous membrane,
- means (2; 32) for measuring the metabolic activity of the biosubstrate disposed against said supporting wall, and
- a main light source (23, 40) disposed appropriately to illuminate the biosubstrate through the supporting wall.

2. Biological sensor according to Claim 1, characterised in that opposite said window (7), the channelling means (1) comprises an opening sealed by a tight cap (8) which is easily removed.

3. Biological sensor according to Claim 1, characterised in that said window is provided in the wall of a chamber (3), into which a water inlet conduit (4) and a water discharge conduit (5) feed.

4. Biological sensor according to Claim 1, characterised in that the measurement means are means for measuring the chlorophyll fluorescence of the biosubstrate.

5. Biological sensor according to Claim 3, characterised in that said measuring means comprise a housing (21) provided with an opening (22) arranged to face the window (7), a light detector (25) located in said opening and provided with an optical filter (26) for measurement of the fluorescence of the biosubstrate, an associated light source (24) for creation of a modulated illumination of the biosubstrate, and processing means for synchronous demodulation of the signal supplied by the detector.

6. Biological sensor according to Claim 3, characterised in that the main light source is formed from light-emitting diodes (23) positioned in the housing (21) and emitting a wavelength close to 550 nm, the associated light source is formed from diodes emitting a wavelength close to 470 nm, the diodes being disposed so that their radiation is directed towards the biosubstrate (11), and the optical filter (26) is a filter which passes wavelengths greater than about 650 nm.

7. Biological sensor according to Claim 1, characterised in that the supporting wall (36) is made of a material which is permeable to oxygen, but impermeable to water, and the measuring means are means (32) for measuring the quantity of oxygen generated by photosynthesis of the biosubstrate and passing through said supporting wall, said measuring means (32) comprising an envelope (33) surrounding an electrode holder (34) and having between them an annular gap (35) filled with electrolyte, said electrode holder bearing on its side an anode (38) and on its front face located close to the supporting wall (36) a cathode (37), which is transparent to light rays, the light source (40) being located in said electrode holder.

8. Method of detecting pollution in a circulating flow of water, characterised in that a chlorophyll biosubstrate (11, 41) is positioned in a window (7, 42) disposed in the wall of a channelling means (1, 31), through which said water passes, said chlorophyll biosubstrate being held between a porous membrane (10, 39) directly communicating with the interior of the channelling means and a transparent supporting wall (12, 36), measuring means (2, 32) for measuring the metabolic activity of the biosubstrate are disposed on the side of said supporting wall opposite the interior of the channelling means, said biosubstrate is illuminated via said supporting wall, and the metabolic activity of the biosubstrate is permanently measured, whereby variations in the signals provided by said measuring means are representative of the pollution status of the water.

9. Method according to Claim 8, characterised in that the biosubstrate is illuminated, on the one hand, by a radiation with a wavelength of close to 550 nm in conditions simulating natural light, and on the other hand by a time-modulated radiation with a wavelength of close to 470 nm in order to induce modulated fluorescence of the biosubstrate, this fluorescence is measured by a light detector (25) provided with a filter (26) blocking wavelengths less than about 650 nm, and synchronous demodulation of the signal provided by the detector (25) is then effected in order to determine the level of fluorescence of the biosubstrate, which is representative of its metabolic activity.

10. Method according to Claim 8, characterised in that the supporting wall is made of a material which is permeable to oxygen, but impermeable to water, and for determination of the metabolic activity of the biosubstrate, the variations in time of the quantity of oxygen generated by photosynthesis of the biosubstrate and passing through said supporting wall are measured.
